# EUROPEAN PATENT APPLICATION

(11) **EP 4 317 455 A1**
(43) Date of publication of application: **07.02.2024**
(21) Application number: 22775999.0
(22) Date of filing: 21.03.2022
(51) Int. Cl.: C12Q 1/686

(54) **TARGET NUCLEIC ACID AMPLIFICATION METHOD USING GUIDE PROBE AND CLAMPING PROBE AND COMPOSITION FOR AMPLIFYING TARGET NUCLEIC ACID COMPRISING SAME**

(30) Priority: 22.03.2021 KR 20210036727
(71) Applicant: HLB Panagene Co., Ltd, Daejeon 34027 (KR)
(72) Inventor: PARK, Jaejin, Seoul 01883 (KR); KIM, Minseo, Sejong 30150 (KR); NAM, Hyo Joo, Suwon-si, Gyeonggi-do 16334 (KR); LEE, Young, Daejeon 34140 (KR)
(74) Representative: Mewburn Ellis LLP
(86) International application number: PCT/KR2022/003869
(87) International publication number: WO 2022/203297

(57) **Abstract**

The present invention relates to: a target nucleic acid amplification method capable of amplifying a very low concentration of a target nucleic acid with high specificity; and a composition for amplifying a target nucleic acid by using same and, more specifically, to: a method for producing an amplicon of a target nucleic acid with high specificity by using, when a target nucleic acid is present, a guide probe binding to the target nucleic acid, a partial primer capable of amplifying the target nucleic acid by binding to the guide probe, and a clamping probe that inhibits the amplification of nucleic acids other than the target nucleic acid; and a composition for a polymerase chain reaction (PCR) therefor. The target nucleic acid amplification method according to the present invention is advantageous in that a target nucleic acid present at a very low concentration can be amplified using a guide probe which binds to all hybridizable nucleic acids present in a sample and helps a partial primer bind to a target nucleic acid detection site, nucleic acids other than the target nucleic acid differ in amplification rate due to the sequence specificity of the partial primer, and the target nucleic acid can be detected with high specificity since the amplification of nucleic acids other than the target nucleic acid is suppressed by a clamping probe, and thus is effective in molecular diagnosis, prenatal diagnosis, early diagnosis, cancer diagnosis, heredity-related diagnosis, genetic trait diagnosis, diagnosis of infectious bacteria, identification of drug-resistant bacteria, forensic medicine, classification of the species of organisms, and the like.

## Description

### [Technical Field]

The present invention relates to a method of amplifying a target nucleic acid capable of amplifying a very low concentration of target nucleic acid with high specificity and a composition for amplifying a target nucleic acid using the same, and more particularly to a method of producing an amplicon of a target nucleic acid with high specificity, which uses, when a target nucleic acid is present, a guide probe binding to the target nucleic acid, a partial primer capable of amplifying the target nucleic acid by binding to the guide probe, and a clamping probe that inhibits amplification of other nucleic acids except the target nucleic acid, and a polymerase chain reaction (PCR) composition for performing the method.

### [Background Art]

The genetic information of all living organisms on earth is the source of unique properties of each individual, which is recorded in the sequence in which bases such as adenine (A), guanine (G), cytosine (C), and thymine (T) or uracil (U) are arranged in a nucleic acid (DNA or RNA) material. Therefore, revealing or confirming the sequence of such bases (nucleotide sequence) may be a process for identifying the properties of living organisms and understanding inherent metabolic mechanisms thereof.

Recently, molecular diagnostics for confirming the presence or properties of living organisms through a method of detecting or identifying a specific nucleotide sequence of the organism has been widely used. Representative examples of molecular diagnostics used medically include detecting gene mutations related to human cancer, detecting pathogens that cause infectious diseases that may occur in humans, etc., and detecting harmful microorganisms present in foods also falls within the range of molecular diagnostics.

Most of various molecular diagnosis techniques for specifically identifying a specific nucleotide sequence adopt polymerase chain reaction (PCR) using a nucleic acid polymerase (DNA polymerase). Polymerase chain reaction is carried out using a composition including a pair of primers capable of specifically hybridizing to a target nucleic acid including a specific nucleotide sequence and a thermo-stable nucleic acid polymerase capable of causing polymerase chain reaction using the target nucleic acid as a template from the primers, and a thermal cycler configured to stepwisely and repeatedly apply a predetermined temperature to the composition. In addition, molecular diagnosis through polymerase chain reaction utilizes a nucleic acid-binding dye or probe to detect, in real time, a specific nucleotide sequence in a target nucleic acid that is mass-produced (amplified), which is called real-time polymerase chain reaction (PCR) (Higuchi, R. et al., Biotechnology 1992. 10:413-417, Higuchi, R. et al., Biotechnology 1993. 11:1026-1030).

In molecular diagnosis, detecting a gene mutation associated with human cancer requires high sensitivity and high specificity. Since the mutation to be detected is a somatic mutation and is present in a very small amount by being mixed with wild-type DNA, when the target mutation is not accurately recognized among point mutations that do not differ significantly in nucleotide sequences from wild-type genes, insertion/deletion mutations in which nucleotide sequence mutations appear complicated, gene fusion mutations, etc., false-positives may be generated.

Specifically, important requirements for tumor-specific mutation detection tests are (1) sensitivity to detect mutant DNA present at a low concentration in wild-type DNA and (2) specificity to minimize a false-positive rate of misdiagnosing wild-type DNA as mutant DNA.

Various test methods for detecting tumor-specific mutations are being developed and used, and representative examples thereof include direct sequencing, allele-specific PCR (AS-PCR), restriction fragment length polymorphism (RFLP), TaqMan probe method, ARMS (amplification refractory mutation system)-PCR, and the like. However, these methods did not show satisfactory results in view of sensitivity and specificity. Direct sequencing has the highest specificity and thus low false-positive rate, but is disadvantageous in that detection is possible only when at least 20 to 30% of mutant DNA is present. On the other hand, AS-PCR, RFLP, and TaqMan probe methods have high sensitivity but low specificity and always have the problem of false positives.

Recently, methods with greatly improved sensitivity and specificity, such as a PNA (peptide nucleic acid)-mediated PCR clamping method (Sun, X., et al., 2002. Nat. Biotechnol., 20: 186-189), an LNA (locked nucleic acid)-mediated PCR clamping method (Dominguez, P. L., et al., 2005. Oncogene, 24: 6830-6834), COLD-PCR (co-amplification at lower denaturation temperature PCR) (Li, J., et al., 2008. Nat. Med, 14: 579-584), and the like, have been developed. As such, sequencing capability with high specificity is gradually being emphasized.

ARMS-PCR was developed to address low specificity, which is a disadvantage of AS-PCR, and is based on the principle that PCR is performed only when primers are composed of a sequence perfectly complementary to the sequence of the template, like AS-PCR (Newton, C. R., et al., 1989. Nucl. Acids Res., 17; 2503-2516). However, in order to determine an optimal primer with excellent specificity, there is a disadvantage in that experimental trial and error is required (Drenkard, E., et al., 2000. Plant Physiol. 124: 1483-1492).

Korean Patent No. 10-2020-0066712 discloses a method of enriching the nucleic acid ratio in a specific population by contacting a plurality of blocking oligonucleotides for selective enrichment of a DNA population in a mixed sample including multiple populations, but is disadvantageous because an aliphatic molecule that inhibits amplification reaction is additionally required for the blocking oligonucleotides. Korean Patent No. 10-2019800 discloses a method of detecting the methylation state of a target gene using a difference in Tm (melting temperature) using a reduction probe, but is disadvantageous because gene mutations cannot be detected and only the methylation state may be detected.

Accordingly, the present inventors have made great efforts to develop a method capable of drastically increasing the specificity of amplification of a target nucleic acid present at a very low concentration, and thus ascertained that, when PCR is performed as a method of producing an amplicon using a guide probe including a sequence capable of hybridizing with a region of a target nucleic acid other than a detection region and a sequence not hybridizing with the target nucleic acid, a partial primer including a sequence complementary to the sequence not hybridizing with the target nucleic acid in the guide probe and a sequence complementary to the detection region of the target nucleic acid, a clamping probe that inhibits amplification of other nucleic acids except the target nucleic acid, and a specific primer capable of amplifying the target nucleic acid by pairing with the partial primer, a very low concentration of target nucleic acid may be detected with high specificity, thus culminating in the present invention.

The above information described in the background section is only for improving the understanding of the background of the present invention, and it does not include information forming the prior art known to those of ordinary skill in the art to which the present invention pertains.

### [Disclosure]

It is an object of the present invention to provide a method of amplifying a very low concentration of target nucleic acid with high specificity.

It is another object of the present invention to provide a polymerase chain reaction (PCR) composition for amplifying a target nucleic acid capable of amplifying a very low concentration of target nucleic acid with high specificity.

In order to accomplish the above objects, the present invention provides a method of amplifying a target nucleic acid, including (a) amplifying an isolated nucleic acid by performing polymerase chain reaction (PCR) in the presence of i) a guide probe including a sequence capable of hybridizing with a region of a target nucleic acid other than a detection region and a sequence not hybridizing with the target nucleic acid, ii) a partial primer including a sequence complementary to the sequence not hybridizing with the target nucleic acid in the guide probe and a sequence complementary to the detection region of the target nucleic acid, iii) a clamping probe that inhibits amplification of other nucleic acids except the target nucleic acid, and iv) a specific primer capable of amplifying the target nucleic acid by pairing with the partial primer and (b) determining the presence or absence of an amplicon.

In addition, the present invention provides a PCR composition for amplifying a target nucleic acid, including i) a guide probe including a sequence capable of hybridizing with a region of a target nucleic acid other than a detection region and a sequence not hybridizing with the target nucleic acid, ii) a partial primer including a sequence complementary to the sequence not hybridizing with the target nucleic acid in the guide probe and a sequence complementary to the detection region of the target nucleic acid, iii) a clamping probe that inhibits amplification of other nucleic acids except the target nucleic acid, and iv) a specific primer capable of amplifying the target nucleic acid by pairing with the partial primer.

### [Description of Drawings]

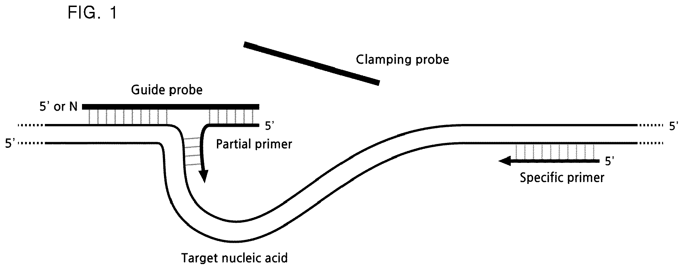

FIG. 1 shows a guide probe, a clamping probe, a partial primer, and a specific primer, which are components necessary for an embodiment of the present invention, and a hybridization relationship between the components;

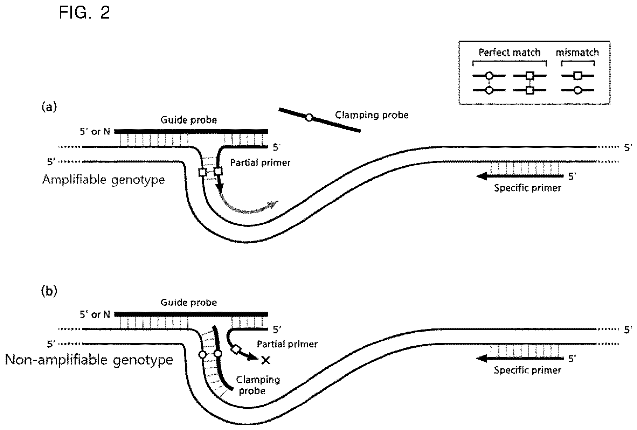

FIG. 2 shows a process of inducing a difference in amplification efficiency based on a difference in nucleotide sequence between a target nucleic acid and a non-target nucleic acid according to the present invention, in which, in the presence of a target nucleic acid in (a), when a partial primer that hybridizes with a portion of a guide probe is close to the target amplification region of the target nucleic acid by the guide probe that hybridizes with a specific region of the target nucleic acid, a portion of the 3' end of the partial primer is complementary to the nucleotide sequence of the target amplification region of the target nucleic acid, resulting in hybridization and extension amplification, in which binding of the guide probe and the target nucleic acid and binding of the guide probe and the partial primer hybridize first due to high Tm, binding of the partial primer and the target nucleic acid hybridizes later due to low Tm because the complementary sequence is short, and binding of the partial primer and the target nucleic acid is not hindered due to low complementarity between the clamping probe and the nucleotide sequence of the target nucleic acid, and also, in the presence of a non-target nucleic acid having a partial nucleotide sequence different from the target nucleic acid in (b), even when the guide probe hybridizes with a specific region of the non-target nucleic acid and thus the partial primer hybridizing with a portion of the guide probe is close to the non-target nucleic acid, complementarity between a portion of the 3' end of the partial primer and the nucleotide sequence of the non-target nucleic acid is low and the clamping probe hybridizes to the non-target nucleic acid, so that binding of the partial primer and the non-target nucleic acid is hindered, resulting in inhibited extension amplification;

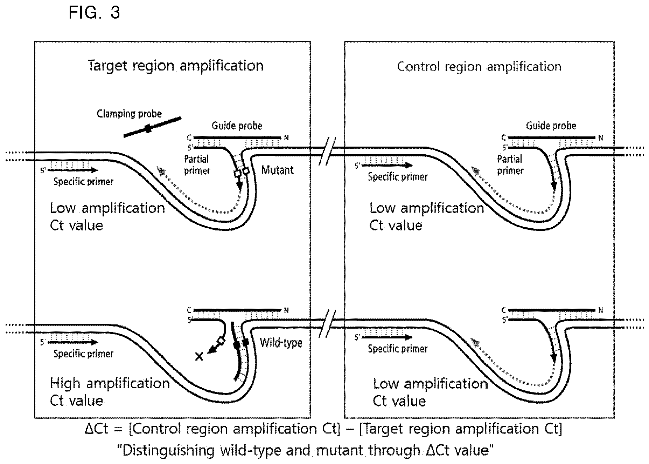

FIG. 3 is a conceptual view showing a process of detecting a specific mutation by distinguishing the same from a wild-type according to an embodiment of the present invention;

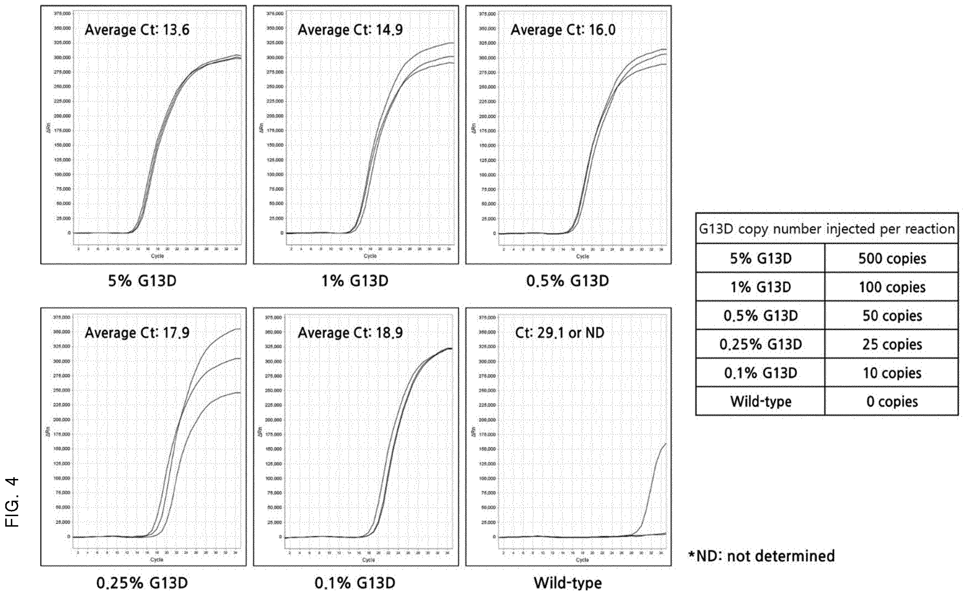

FIG. 4 shows results of detecting G13D mutation of the KRAS gene in the human genome with high sensitivity and specificity according to an embodiment of the present invention, in which, when using a partial primer configured such that a portion of the 3' end hybridizes with the genotype of G13D mutation and a clamping probe configured to hybridize with a wild-type, high amplification efficiency is obtained in the presence of G13D mutation, yielding a relatively low Ct value, whereas when a wild-type is present alone, low amplification efficiency results in a relatively high Ct value or no amplification, whereby G13D mutation, which is present in a very small amount of 0.1%, may be detected by comparing Ct values;

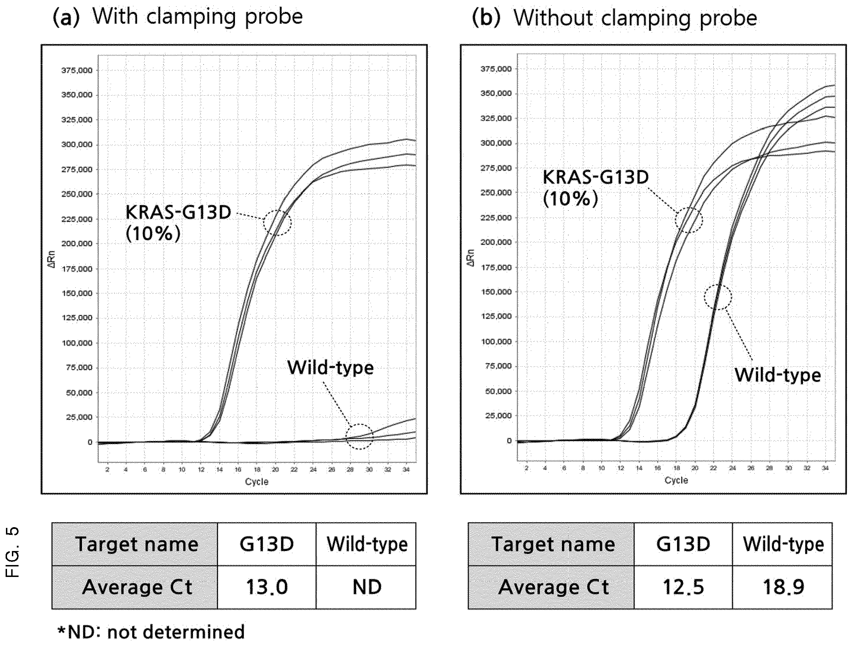

FIG. 5 shows a comparison of results of detecting G13D mutation of the KRAS gene in the human genome in an embodiment of the present invention (a) and under the condition without a clamping probe (b), in which, when a partial primer configured such that a portion of the 3' end hybridizes with the genotype of G13D mutation and a clamping probe that hybridizes to a wild-type are used together, a difference in Ct value between the condition with G13D mutation and the condition with only the wild-type is very large, whereas the difference in Ct value between the condition with G13D mutation and the condition with the wild-type is small under the condition without the clamping probe;

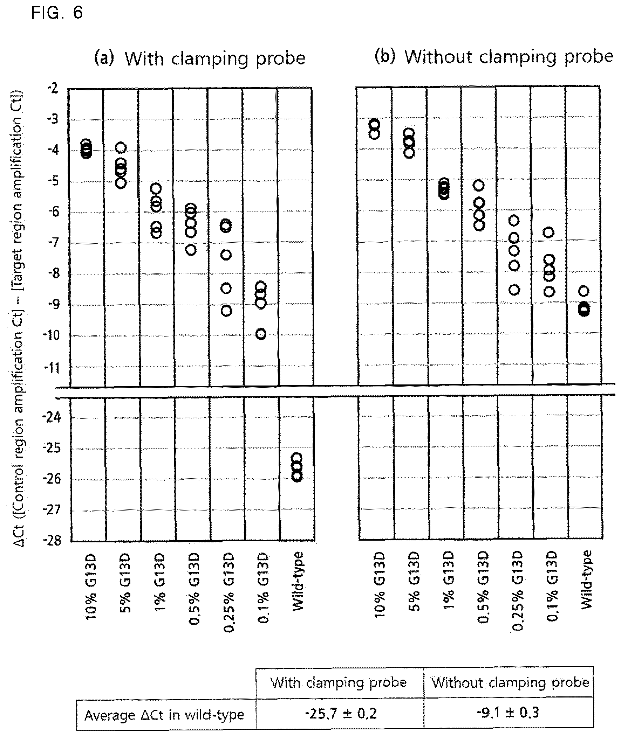

FIG. 6 shows a comparison of results of detecting G13D mutation of the KRAS gene in the human genome in an embodiment of the present invention (a) and under the condition without a clamping probe (b), in which when a partial primer configured such that a portion of the 3' end hybridizes with the genotype of G13D mutation and a clamping probe that hybridizes to a wild-type are used together, G13D mutation present in a very small amount of 0.1% may be detected based on a ΔCt value representing a difference between the control region amplification Ct value and the target region amplification Ct value, whereas G13D mutation present in an amount of 0.5% may be detected based on the ΔCt value under the condition without the clamping probe, but G13D mutation present in a lower amount (0.1-0.25%) is difficult to distinguish from the wild-type; and

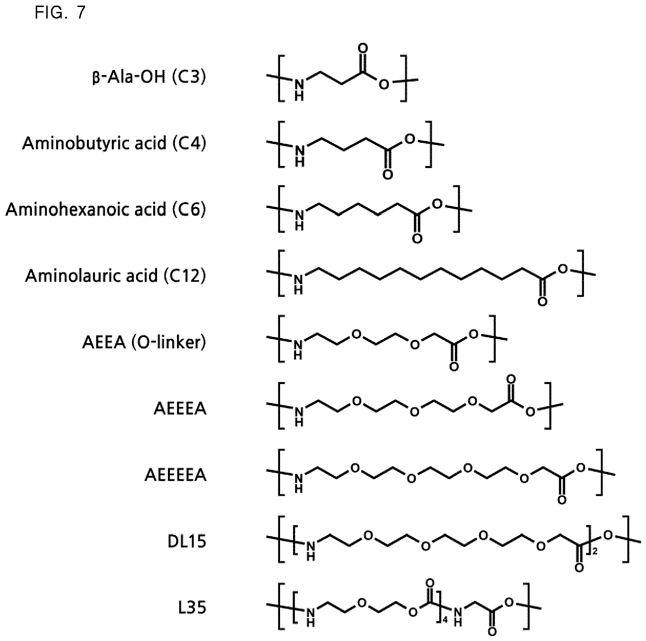

FIG. 7 shows examples of a linker that may be included in the guide probe of the present invention.

### [Mode for Invention]

Unless otherwise defined, all technical and scientific terms used herein have the same meanings as typically understood by those skilled in the art to which the present invention belongs. In general, the nomenclature used herein is well known in the art and is typical.

The present invention is intended to confirm whether a very low concentration of target nucleic acid may be detected using a guide probe, a clamping probe, and a partial primer.

In an embodiment of the present invention, in order to specifically amplify and detect G13D mutation, which is a GGC>GAC nucleotide sequence mutation located at exon 2, codon 13 of the human KRAS gene, a guide probe capable of specifically hybridizing to a region near codon 13 of a KRAS gene, a clamping probe capable of specifically hybridizing to codon 13 and a nearby region of a wild-type KRAS gene, a partial primer configured such that a portion of the 3' end is able to specifically hybridize to codon 13 and a nearby region of a KRAS gene with G13D mutation, and a specific primer capable of hybridizing with a portion of the nucleotide sequence of the KRAS gene to amplify a target nucleic acid by pairing with the partial primer were designed. Here, a partial nucleotide sequence at the C terminus of the guide probe and a partial nucleotide sequence at the 5' end of the partial primer were allowed to hybridize.

PCR was performed using the guide probe, the clamping probe, the partial primer, and the specific primer to determine whether it is possible to detect a very small amount of G13D mutation in the presence of a wild-type nucleic acid.

Thereby, the fluorescence signal on the amplification curve increased rapidly in the presence of the G13D mutation target nucleic acid, yielding a low Ct value, whereas an increase in the fluorescence signal on the amplification curve did not occur or was very slow in the presence of only the wild-type nucleic acid, yielding a high Ct value (FIG. 4).

Accordingly, an aspect of the present invention pertains to a method of amplifying a target nucleic acid, including:
(a) amplifying an isolated nucleic acid by performing polymerase chain reaction (PCR) in the presence of:
   i) a guide probe including a sequence capable of hybridizing with a region of a target nucleic acid other than a detection region and a sequence not hybridizing with the target nucleic acid,
   ii) a partial primer including a sequence complementary to the sequence not hybridizing with the target nucleic acid in the guide probe and a sequence complementary to the detection region of the target nucleic acid,
   iii) a clamping probe that inhibits amplification of other nucleic acids except the target nucleic acid, and
   iv) a specific primer capable of amplifying the target nucleic acid by pairing with the partial primer, and
(b) determining the presence or absence of an amplicon.

As used herein, the term "target nucleic acid" refers to all types of nucleic acids including a nucleotide sequence of interest to be amplified or detected. The target nucleic acid includes gene sequences derived from various species, subspecies, or variants, or gene mutations within the same species, and examples thereof may include, but are not limited to, all types of DNA including genomic DNA, mitochondrial DNA, and viral DNA, or all types of RNA including mRNA, ribosomal RNA, non-coding RNA, tRNA, viral RNA, etc. The target nucleic acid may hybridize with a guide probe, a partial primer (a portion of 3' end), and a specific primer under conditions for polymerase chain reaction.

As used herein, the term "hybridization" refers to formation of a double-stranded nucleic acid by hydrogen bonding between single-stranded nucleic acids having complementary nucleotide sequences, and is used in a similar sense to annealing. However, in a slightly broader sense, hybridization includes cases where nucleotide sequences between two single strands are perfectly complementary (perfect match) as well as exceptional cases where some nucleotide sequences are not complementary (mismatch).

As used herein, the term "guide probe" is capable of hybridizing with a target nucleic acid and a partial primer at the same time, and helps the 3' end of the partial primer to hybridize with the target nucleotide sequence of the target nucleic acid. Briefly, the sequence not hybridizing with the target nucleic acid in the guide probe may hybridize with the partial primer. The guide probe may be made of any one or a mixture of two or more selected from among all materials (e.g. DNA, RNA, LNA (locked nucleic acid), PNA (peptide nucleic acid), etc.) capable of hybridizing with the target nucleic acid.

As used herein, the term "clamping probe" is capable of hybridizing to a non-target nucleic acid for which amplification is not desired, and prevents the 3' end of the partial primer from hybridizing with a non-target nucleic acid. The clamping probe may be made of any one or a mixture of two or more selected from among all materials (e.g. DNA, RNA, LNA (locked nucleic acid), PNA (peptide nucleic acid), etc.) capable of hybridizing with a non-target nucleic acid.

As used herein, the term "partial primer" is configured such that a portion of the 5' end hybridizes with the guide probe and the 3' end hybridizes with the target nucleotide sequence of the target nucleic acid, resulting in synthetic extension through a nucleic acid polymerase.

In a preferred embodiment of the present invention, the target nucleic acid, the guide probe, and the partial primer mutually hybridize (target nucleic acid-guide probe, guide probe-partial primer, partial primer-target nucleic acid). As the guide probe binds near the target nucleotide sequence of the target nucleic acid, the partial primer that is hybridized is located near the target nucleotide sequence, whereby the 3' end of the partial primer more easily hybridizes to the target nucleotide sequence, thus initiating synthetic extension through a nucleic acid polymerase and target region amplification of the target nucleic acid.

For hybridization in the target nucleic acid-guide probe and the guide probe-partial primer, the complementary portions hybridize first due to high Tm compared to the partial primer-target nucleic acid, and the partial primer-target nucleic acid hybridizes later due to relatively low Tm.

As used herein, the term "specific primer" is capable of hybridizing to a target nucleic acid without the help of the guide probe, leading to synthetic extension through a nucleic acid polymerase.

In the present invention, the guide probe is configured such that the region hybridizing with the target nucleic acid is present at the 5' end or N-terminus and the region hybridizing with the partial primer is present at the 3' end or C-terminus, but the present invention is not limited thereto.

In the present invention, the guide probe may further include a linker between the sequence hybridizing with the target nucleic acid and the sequence not hybridizing with the target nucleic acid.

In the present invention, the linker may be used without limitation, so long as it is a compound not containing a nucleobase, which is present between the sequence hybridizing with the target nucleic acid and the sequence hybridizing with the partial primer and connects the two regions, and is preferably at least one selected from the group consisting of beta-alanine (β-Ala-OH, C3), aminobutyric acid (C4), aminohexanoic acid (C6), aminolauric acid (C12), acetoacetoxyethyl acrylate (AAEA (O-linker)), 2-[2-[2-[2-(amino)ethoxy]ethoxy]ethoxy]acetic acid (AEEEA), AEEEEA, DL15, and L35, but is not limited thereto.

In the present invention, each of the guide probe, the clamping probe, the partial primer, and the specific primer is composed of any one or a combination of two or more selected from among an oligonucleotide, LNA (locked nucleic acid), and PNA (peptide nucleic acid).

More specifically, each of the guide probe and the clamping probe may be made of any one or a combination of two or more selected from among an oligonucleotide, LNA, and PNA.

For example, when the guide probe is 20 bp long, the 10-bp 5' end may be composed of PNA and the 10-bp 3' end may be composed of an oligonucleotide.

Each of the partial primer and the specific primer may be made of any one or a combination of two or more selected from among an oligonucleotide, LNA, and PNA, but the sequence of 5 or more bases at the 3' end may be composed of an oligonucleotide to induce amplification reaction.

In the present invention, the guide probe may be used without limitation, so long as it is a nucleic acid capable of hybridizing with a target nucleic acid and a partial primer, but is preferably PNA with a length of 10 to 500 bases, more preferably PNA with a length of 20 to 150 bases.

In the present invention, the clamping probe may be used without limitation, so long as it is a nucleic acid capable of hybridizing to a non-target nucleic acid, but is preferably PNA with a length of 5 to 500 bases, more preferably PNA with a length of 10 to 100 bases.

In the present invention, the clamping probe may prevent the partial primer from binding to other nucleic acids except the target nucleic acid.

In the present invention, the clamping probe may include the sequence complementary to the detection region of the target nucleic acid in the partial primer, which differs by 1 to 10 bases.

In the present invention, since the clamping probe has a target nucleic acid detection region binding sequence different from the partial primer, it prevents the partial primer from binding to nucleic acids present in the sample other than the target nucleic acid and does not bind to the target nucleic acid.

In the present invention, the guide probe and the clamping probe may hybridize to a strand of the target nucleic acid opposite a strand to which the specific primer hybridizes.

In the present invention, the partial primer may further include a spacer, which is a single-stranded oligonucleotide with a length of 1 to 100 bases, between the sequence complementary to the sequence not hybridizing with the target nucleic acid in the guide probe and the sequence complementary to the detection region of the target nucleic acid.

In the present invention, the spacer is a sequence except the sequence complementary to the guide probe and the sequence complementary to the target nucleic acid included in the partial primer, and as necessary, the spacer may function to perform detection with the same probe even when the target nucleic acid sequence is changed using a probe capable of binding to the spacer.

In the present invention, the spacer and the linker of the guide probe may play a role in controlling binding efficiency of the partial primer located near the target region of the target nucleic acid by the guide probe to the target region. This means that, when the guide probe hybridizes to the target nucleic acid at a position slightly distant from the target region, increasing the length of the spacer and the linker may help the partial primer bind to the target region.

In the present invention, the sequence complementary to the detection region of the target nucleic acid in the partial primer may have a length of 3 to 15 bases.

In the present invention, the amplicon may be 50 bp to 1 kbp in length.

In the present invention, determining the presence or absence of the amplicon may be performed using a nucleic acid-binding dye or probe capable of binding to the amplicon.

In the present invention, the nucleic acid-binding dye may be used without limitation, so long as it is an intercalating material or a DNA minor groove binding material, but is preferably selected from the group consisting of ethidium bromide, SYBR^{®} Green I, SYBR^{®} Gold, EvaGreen, YO-PRO-1, SYTO, BEBO, and BEXTO.

In the present invention, the probe capable of binding to the amplicon may be selected from the group consisting of any one or a combination of two or more selected from among an oligonucleotide, LNA, and PNA.

PNA (peptide nucleic acid) is a DNA mimic in which nucleobases are attached to a peptide backbone rather than a sugar-phosphate backbone, and was first synthesized by Nielsen et al. in 1991. PNA is an artificially synthesized gene recognition material, like LNA (locked nucleic acid) or MNA (morpholino nucleic acid), and is fundamentally composed of a polyamide backbone.

PNA has excellent affinity and selectivity, and has high stability against nucleases, so it is not degraded by existing restriction enzymes. Also, there is an advantage of easy long-term storage by virtue of high thermal/chemical stability.

PNA forms a double strand through hybridization with a natural nucleic acid having a complementary nucleotide sequence. PNA/DNA duplexes are more stable than DNA/DNA duplexes and PNA/RNA duplexes are more stable than DNA/RNA duplexes, when they are the same length. Moreover, PNA has higher ability to detect SNP (single nucleotide polymorphism) than natural nucleic acids because the extent to which duplexes become unstable is large due to single base mismatches.

Specifically, PNA-DNA binding affinity is much stronger than DNA-DNA binding affinity, and the melting temperature (Tm) usually differs by about 15 to 20°C even with one nucleotide mismatch. It is possible to detect nucleotide sequence mutations such as SNP (single nucleotide polymorphism) and In/Del (insertion/deletion) using this difference in binding affinity.

In the present invention, the probe capable of binding to the amplicon may have a nucleotide sequence partially or wholly complementary to any nucleotide sequence and target nucleic acid sequence in the amplicon, and is preferably configured such that a reporter and a quencher are connected at both ends thereof.

In the present invention, signal generation is suppressed when the distance between the reporter and the quencher in the probe is short, but signal intensity increases with an increase in the distance between the reporter and the quencher. In general, when the probe hybridizes with a complementary nucleotide sequence, the distance between the reporter and the quencher becomes the greatest, so that a specific nucleotide sequence may be detected through signal generation or an increase in signal intensity.

In the present invention, the reporter may be at least one fluorescent material selected from the group consisting of fluorescein, fluorescein chlorotriazinyl, Rhodamine Green, Rhodamine Red, tetramethylrhodamine, FITC, Oregon Green, Alexa Fluor, FAM, JOE, ROX, HEX, Texas Red, TET, TRITC, TAMRA, cyanine-based dyes, and thiadicarbocyanine dyes.

In the present invention, the quencher may be at least one selected from the group consisting of Dabcyl, TAMRA, Eclipse, DDQ, QSY, Blackberry Quencher, Black Hole Quencher, Qxl, Iowa black FQ, Iowa Black RQ, and IRDye QC-1.

In a preferred embodiment of the present invention, detection of the amplicon using the nucleic acid polymerase is performed through real-time polymerase chain reaction (real-time PCR). Here, an amplification curve with an increase in the amplicon is created to obtain a Ct (threshold cycle) value, but the present invention is not limited thereto. The method using the Ct value is based on a principle in which the number of cycles reaching the threshold is reduced and the Ct value is measured less by early increasing the amount of signal generated by the detection probe with earlier production and increase in the amplicon due to the presence of the target nucleic acid (target nucleotide sequence) in the sample.

In the present invention, the isolated nucleic acid may be used without limitation, so long as it is a nucleic acid without other materials capable of inhibiting polymerase chain reaction, and is preferably isolated from a sample of a subject, but is not limited thereto.

Another aspect of the present invention pertains to:
a PCR composition for amplifying a target nucleic acid, including:
i) a guide probe including a sequence capable of hybridizing with a region of a target nucleic acid other than a detection region and a sequence not hybridizing with the target nucleic acid,
ii) a partial primer including a sequence complementary to the sequence not hybridizing with the target nucleic acid in the guide probe and a sequence complementary to the detection region of the target nucleic acid,
iii) a clamping probe that inhibits amplification of other nucleic acids except the target nucleic acid, and
iv) a specific primer capable of amplifying the target nucleic acid by pairing with the partial primer.

As used herein, the term "sample" includes various samples, and preferably, a biological sample is analyzed using the method of the present invention. More preferably, the sample is a sample mixed with virus species or a sample of a subject infected with the virus (e.g. human, mammal, fish, etc.), and biological samples of plant, animal, human, fungal, bacterial, and viral origin may be analyzed. When analyzing a sample of mammalian or human origin, the sample may originate from a specific tissue or organ. Representative examples of tissues include connective, skin, muscle, or nerve tissues. Representative examples of organs include the eyes, brain, lungs, liver, spleen, bone marrow, thymus, heart, lymph, blood, bone, cartilage, pancreas, kidneys, gallbladder, stomach, small intestine, testicles, ovaries, uterus, rectum, nervous system, glands, and internal blood vessels. The biological sample to be analyzed includes any cell, tissue, or fluid from a biological source, or any other medium that may well be analyzed by the present invention, and includes samples obtained from humans, animals, or foods prepared for consumption to humans or animals. Moreover, the biological samples to be analyzed include fluid samples, such as blood, serum, plasma, lymph, breast milk, urine, feces, ocular fluid, saliva, semen, brain extracts (e.g. brain fragments), spinal fluid, appendix, spleen, and tonsil tissue extracts, but are not limited thereto.

Still another aspect of the present invention pertains to a kit for detecting a target nucleic acid including the composition.

In the present invention, the kit may optionally include a reagent necessary for carrying out target nucleic acid amplification reaction (e.g. polymerase chain reaction) such as a buffer, a DNA polymerase, a DNA polymerase cofactor, and deoxyribonucleotide-5-triphosphate (dNTP). Optionally, the kit of the present invention may also include various oligonucleotide molecules, reverse transcriptases, various buffers and reagents, and antibodies that inhibit DNA polymerase activity. Moreover, the optimal amount of the reagent used for particular reaction of the kit may be easily determined by those skilled in the art having learned the disclosure herein. Typically, the device of the present invention may be manufactured in a separate package or compartment containing the aforementioned components.

In one embodiment, the kit may include a compartmentalized carrier means accommodating a sample, a container including a reagent, a container including a guide probe, a clamping probe, and a primer, and a container including a probe for detecting the amplicon.

The carrier means is suitable for accommodating one or more containers, such as bottles and tubes, and each container contains independent components for use in the method of the present invention. In the context of the present invention, the agent necessary for the container may be readily dispensed by those skilled in the art.

A better understanding of the present invention may be obtained through the following examples. These examples are merely set forth to illustrate the present invention, and are not to be construed as limiting the scope of the present invention, as will be obvious to those skilled in the art.

Example 1. High-sensitivity amplification and detection of KRAS gene with G13D mutation in human genome

### 1.1 Construction of primer and probe

The human KRAS gene encodes a GTP-based switch protein that functions in the body's signaling system, and specific mutations of the KRAS gene are known to be associated with cancer (Waters, A. M. and Der, C. J. 2018. Cold Spring Harb. Perspect. Med. 8:a031435). The GGC>GAC nucleotide sequence mutation located at exon 2, codon 13 of the human KRAS gene described above is referred to as a G13D mutation.

In order to specifically amplify and detect the G13D mutation of the KRAS gene through a novel method of amplifying a target nucleic acid according to the present invention, a guide probe 1 capable of specifically hybridizing to a region near codon 13 of a KRAS gene, a clamping probe 1 capable of hybridizing to codon 13 and a nearby region of a wild-type KRAS gene without G13D mutation, a partial primer 1, a portion of the 3' end of which is capable of specifically hybridizing to codon 13 and a nearby region of a KRAS gene with G13D mutation, and a specific primer 1 capable of hybridizing with a portion of the nucleotide sequence of the KRAS gene to amplify the target nucleic acid by pairing with the partial primer 1 were designed. Here, a partial nucleotide sequence at the C-terminus of the guide probe 1 and a partial nucleotide sequence at the 5' end of the partial primer 1 were allowed to hybridize. Moreover, a detection probe 1 was designed and constructed to detect an amplicon resulting from the designed guide probe and primers (PANAGENE Inc., South Korea). The nucleotide sequences of the primers and probes are shown in Tables 1 and 2 below.

**[Table 1]**

| Primer sequences | | |
|---|---|---|
| Name | Sequence number | Nucleotide sequence (5' end --> 3' end) |
| Partial primer 1 | SEQ ID NO: 1 | GCGTGACTCG T*CTACGTCA* |
| Specific primer 1 | SEQ ID NO: 2 | GTGACATGTTCTAATATAGTCACATTTTC |

**[Table 2]**

| Probe sequences | | |
|---|---|---|
| Name | Sequence number | Nucleotide sequence (N terminus --> C terminus) |
| Guide probe 1 | SEQ ID NO: 3 | [K]-*AAGGCACTCTTGC*-[L35 linker]-CGAGTCACGC |
| Clamping probe 1 | SEQ ID NO: 4 | [K]-CTACGCCACCAGCTC |
| Detection probe 1 | SEQ ID NO: 5 | [Dabcyl]-TAAACTTGTGGTAG-[O linker]-[K]-[FAM] |

The partial primer 1 was made of a single-stranded oligonucleotide with a length of 19 bases, the underlined nucleotide sequence was complementary to a portion of the nucleotide sequence of the guide probe 1, and the italicized nucleotide sequence was complementary to codon 13 and a nearby nucleotide sequence of the KRAS gene with G13D mutation.

The guide probe 1 was made of PNA with a length of 23 bases, the italicized nucleotide sequence was complementary to the nucleotide sequence near codon 13 of the KRAS gene, and the underlined nucleotide sequence was complementary to a portion of the nucleotide sequence of the partial primer 1. Also, [K (lysine)] was attached to the N-terminus of the guide probe 1, and an L35 linker (PANAGENE Inc., South Korea) was connected between the region complementary to the nucleotide sequence of the KRAS gene and the region complementary to a portion of the nucleotide sequence of the partial primer 1.

The clamping probe 1 was made of PNA with a length of 15 bases, and [K (lysine)] was attached to the N-terminus.

The detection probe 1 was made of PNA with a length of 14 bases, and was configured such that [Dabcyl], which is a quencher, was attached to the N-terminus, [FAM], which is a reporter, was attached to the C-terminus, and [O linker] (PANAGENE Inc., South Korea) and [K (lysine)] were connected between PNA and [FAM]. When the detection probe 1 hybridizes to an amplicon having a complementary nucleotide sequence, the quencher and the reporter are the farthest apart, and as such, the signal (fluorescence) value from the reporter is maximized, and the amplicon may be detected by measuring the signal.

The partial primer 1 alone has difficulty hybridizing to the target region under polymerase chain reaction (PCR) conditions because the nucleotide sequence complementary to the nucleotide sequence of the target region in the KRAS gene has a length of only 8 bases. In the presence of the guide probe 1 that is hybridized near the target region, a portion of the nucleotide sequence of the guide probe 1 and a portion of the nucleotide sequence of the partial primer 1 may bind and thus the partial primer 1 may hybridize easily to the target region.

The partial primer 1 has a length of only 8 bases hybridizable with the target nucleic acid sequence of the KRAS gene, which is shorter than typical primers with a length of 18 to 30 bases hybridizable with the target nucleic acid. Here, if at least one non-complementary (mismatch) nucleotide sequence is present between the partial primer 1 and the region hybridizing with the target nucleic acid, the effect of the non-complementary nucleotide sequence on hybridization efficiency is great compared to when using typical primers, ultimately making it possible to more efficiently detect minute changes in the nucleotide sequence than typical primers.

In addition, if hybridization efficiency is decreased due to the presence of at least one non-complementary nucleotide sequence between the partial primer 1 and the codon 13 and the nearby nucleotide sequence of the wild-type KRAS gene without G13D mutation, the clamping probe 1 hybridizes to the codon 13 and the nearby nucleotide sequence of the wild-type KRAS gene, further hindering hybridization of the partial primer 1, thereby making it possible to detect subtle changes in the nucleotide sequence with higher sensitivity than when not using the clamping probe.

### 1.2 Production and verification of amplicon

In order to produce the intended amplicon under conditions in which about 10,000 copies of a wild-type KRAS gene target nucleic acid were present, conditions in which about 10,000 copies of a wild-type KRAS gene target nucleic acid and about 500 copies of a KRAS gene target nucleic acid with G13D mutation were mixed (5%), conditions in which about 10,000 copies of a wild-type KRAS gene target nucleic acid and about 100 copies of a KRAS gene target nucleic acid with G13D mutation were mixed (1%), conditions in which about 10,000 copies of a wild-type KRAS gene target nucleic acid and about 50 copies of a KRAS gene target nucleic acid with G13D mutation were mixed (0.5%), conditions in which about 10,000 copies of a wild-type KRAS gene target nucleic acid and about 25 copies of a KRAS gene target nucleic acid with G13D mutation were mixed (0.25%), or conditions in which about 10,000 copies of a wild-type KRAS gene target nucleic acid and about 10 copies of a KRAS gene target nucleic acid with G13D mutation were mixed (0.1%), a PCR composition including 10 pmoles of guide probe 1, 5 pmoles of clamping probe 1, 10 pmoles of partial primer 1, 4 pmoles of specific primer 1, and 3 pmoles of detection probe 1 was prepared.

The PCR composition included not only a nucleic acid polymerase (DNA polymerase) for typical use in PCR but also components such as a buffer, deoxyribonucleotide-5-triphosphate (dNTP), potassium chloride (KCl), magnesium chloride (MgCl₂), a surfactant, etc.

Temperature control for PCR was performed using a general thermal cycler, and after initial denaturation [95°C, 5 minutes], as a first stage of 15 cycles, denaturation [95°C, 20 seconds] - annealing [63°C, 20 seconds] - extension [72°C, 20 seconds] was performed, after which as a second stage of 35 cycles, denaturation [95°C, 10 seconds] - measurement [50°C, 10 seconds] - denaturation [95°C, 20 seconds] - annealing [58°C, 20 seconds] - extension [72°C, 20 seconds] was performed. In the measurement step of the second stage, an amplification curve was created by measuring the fluorescence value of the FAM channel at every cycle.

Thereby, an increase in fluorescence signal on the amplification curve was clearly observed under all conditions in which the KRAS target nucleic acid with G13D mutation was mixed, whereas an increase in fluorescence signal on the amplification curve was not observed or very weak under the conditions in which only the wild-type KRAS target nucleic acid was present. Specifically, average Ct values of 13.6, 14.9, 16.0, 17.9, and 18.9 were measured under the mixed conditions of about 500, 100, 50, 25, and 10 copies of the target nucleic acid with G13D mutation, respectively, whereas when only the wild-type was present, the Ct value was not calculated (ND; not determined) or a relatively high Ct value of 29.1 was measured, indicating that G13D mutation present in a very small amount could be specifically identified based on a difference in Ct values (FIG. 4).

### Example 2. Confirmation of detection limit of KRAS gene with G13D mutation in human genome

### 2.1 Construction of primer and probe

In order to specifically amplify and detect the G13D mutation of the KRAS gene through the novel method of amplifying the target nucleic acid according to the present invention, a guide probe 1 capable of specifically hybridizing to a region near codon 13 of a KRAS gene, a clamping probe 1 capable of hybridizing to codon 13 and a nearby region of a wild-type KRAS gene without G13D mutation, a partial primer 1, a portion of the 3' end of which is capable of specifically hybridizing to codon 13 and a nearby region of a KRAS gene with G13D mutation, and a specific primer 1 capable of hybridizing with a portion of the nucleotide sequence of the KRAS gene to amplify the target nucleic acid by pairing with the partial primer 1 were designed. Here, a partial nucleotide sequence at the C-terminus of the guide probe 1 and a partial nucleotide sequence at the 5' end of the partial primer 1 were allowed to hybridize. Also, a detection probe 1 was designed and constructed to detect an amplicon resulting from the designed guide probe and primers (PANAGENE Inc., South Korea).

In addition, in order to specifically amplify and detect the KRAS gene in the sample regardless of the presence or absence of G13D mutation in the KRAS gene, a guide probe 2 capable of specifically hybridizing to exon 6 of the KRAS gene, a partial primer 2, a portion of the 3' end of which is capable of specifically hybridizing to exon 6 of the KRAS gene, and a specific primer 2 capable of hybridizing with a portion of the nucleotide sequence of exon 6 of the KRAS gene to amplify the target nucleic acid by pairing with the partial primer 2 were designed. Here, a partial nucleotide sequence at the C terminus of the guide probe 2 and a partial nucleotide sequence at the 5' end of the partial primer 2 were allowed to hybridize. Also, a detection probe 2 was designed and constructed to detect an amplicon resulting from the designed guide probe and primers (PANAGENE Inc., South Korea). The nucleotide sequences of the primers and probes are shown in Tables 3 and 4 below.

**[Table 3]**

| Primer sequences | | |
|---|---|---|
| Name | Sequence number | Nucleotide sequence (5' end --> 3' end) |
| Partial primer 1 | SEQ ID NO: 1 | GCGTGACTCGT*CTACGTCA* |
| Specific primer 1 | SEQ ID NO: 2 | GTGACATGTTCTAATATAGTCACATTTTC |
| Partial primer 2 | SEQ ID NO: 6 | GTCGTCCGTG*CCTTCCACA* |
| Specific primer 2 | SEQ ID NO: 7 | TGTAGGGCATTTCTGATGTGACTC |

**[Table 4]**

| Probe sequences | | |
|---|---|---|
| Name | Sequence number | Nucleotide sequence (N terminus --> C terminus) |
| Guide probe 1 | SEQ ID NO: 3 | [K]-*AAGGCACTCTTGC*-[L35 linker]-CGAGTCACGC |
| Clamping probe 1 | SEQ ID NO: 4 | [K]-CTACGCCACCAGCTC |
| Detection probe 1 | SEQ ID NO: 5 | [Dabcyl]-TAAACTTGTGGTAG-[O linker]-[K]-[FAM] |
| Guide probe 2 | SEQ ID NO: 8 | [K]-*AGTGTCATCTTGCCT*-[L35 linker]-CACGGACGAC |
| Detection probe 2 | SEQ ID NO: 9 | [Dabcyl]-CAGCAGTAAATCT-[O linker]-[K]-[HEX] |

The partial primer 1 was made of a single-stranded oligonucleotide with a length of 19 bases, the underlined nucleotide sequence was complementary to a portion of the nucleotide sequence of the guide probe 1, and the italicized nucleotide sequence was complementary to codon 13 and a nearby nucleotide sequence of the KRAS gene with G13D mutation.

The partial primer 2 was made of a single-stranded oligonucleotide with a length of 19 bases, the underlined nucleotide sequence was complementary to a portion of the nucleotide sequence of the guide probe 2, and the italicized nucleotide sequence was complementary to a portion of the nucleotide sequence of exon 6 of the KRAS gene.

The guide probe 1 was made of PNA with a length of 23 bases, the italicized nucleotide sequence was complementary to the nucleotide sequence near codon 13 of the KRAS gene, and the underlined nucleotide sequence was complementary to a portion of the nucleotide sequence of the partial primer 1. Also, [K (lysine)] was attached to the N-terminus of the guide probe 1, and an L35 linker (PANAGENE Inc., South Korea) was connected between the region complementary to the nucleotide sequence of the KRAS gene and the region complementary to a portion of the nucleotide sequence of the partial primer 1.

The guide probe 2 was made of PNA with a length of 25 bases, the italicized nucleotide sequence was complementary to a portion of the nucleotide sequence of exon 6 of the KRAS gene, and the underlined nucleotide sequence was complementary to a portion of the nucleotide sequence of the partial primer 2. Also, [K (lysine)] was attached to the N-terminus of the guide probe 2, and an L35 linker (PANAGENE Inc., South Korea) was connected between the region complementary to the nucleotide sequence of the KRAS gene and the region complementary to a portion of the nucleotide sequence of the partial primer 2.

The clamping probe 1 was made of PNA with a length of 15 bases, and [K (lysine)] was attached to the N-terminus.

The detection probe 1 was made of PNA with a length of 14 bases, and was configured such that [Dabcyl], which is a quencher, was attached to the N-terminus, [FAM], which is a reporter, was attached to the C-terminus, and [O linker] (PANAGENE Inc., South Korea) and [K (lysine)] were connected between PNA and [FAM].

The detection probe 2 was made of PNA with a length of 13 bases, and was configured such that [Dabcyl], which is a quencher, was attached to the N-terminus, [HEX], which is a reporter, was attached to the C-terminus, and [O linker] (PANAGENE Inc., South Korea) and [K (lysine)] were connected between PNA and [HEX].

When the detection probe 1 and the detection probe 2 hybridize to an amplicon having a complementary nucleotide sequence, the quencher and the reporter are the farthest apart, and as such, the signal (fluorescence) value from the reporter is maximized, and the amplicon may be detected by measuring the signal.

The partial primer 1 alone has difficulty hybridizing to the target region under PCR conditions because the nucleotide sequence complementary to the nucleotide sequence of the target region in the KRAS gene has a length of only 8 bases. In the presence of the guide probe 1 that is hybridized near the target region, a portion of the nucleotide sequence of the guide probe 1 and a portion of the nucleotide sequence of the partial primer 1 may bind and thus the partial primer 1 may hybridize easily to the target region.

The partial primer 1 has a length of only 8 bases hybridizable with the target nucleic acid sequence of the KRAS gene, which is shorter than typical primers having a length of 18 to 30 bases hybridizable with the target nucleic acid. Here, if at least one non-complementary (mismatch) nucleotide sequence is present between the partial primer 1 and the region hybridizing with the target nucleic acid, the effect of the non-complementary nucleotide sequence on hybridization efficiency is great compared to when using typical primers, ultimately making it possible to more efficiently detect minute changes in the nucleotide sequence than typical primers.

In addition, if hybridization efficiency is lowered due to the presence of at least one non-complementary nucleotide sequence between the partial primer 1 and the codon 13 and the nearby nucleotide sequence of the wild-type KRAS gene without G13D mutation, the clamping probe 1 hybridizes to the codon 13 and the nearby nucleotide sequence of the wild-type KRAS gene, further hindering hybridization of the partial primer 1, thereby making it possible to detect subtle changes in the nucleotide sequence with higher sensitivity than when not using the clamping probe.

The partial primer 2 alone has difficulty hybridizing to the target region under PCR conditions because the nucleotide sequence complementary to the nucleotide sequence of the target region in the KRAS gene has a length of only 9 bases. In the presence of the guide probe 2 that is hybridized near the target region, a portion of the nucleotide sequence of the guide probe 2 and a portion of the nucleotide sequence of the partial primer 2 may bind and thus the partial primer 2 may hybridize easily to the target region.

The 3' end of the partial primer 2 hybridizes with a portion of the nucleotide sequence of exon 6 of the KRAS gene, which may occur regardless of the presence or absence of the G13D mutation in the KRAS gene. In consideration thereof, amplification through the partial primer 2 may be used as a control.

If the G13D mutation of the KRAS gene is not present in the sample, amplification through the partial primer 1 (target amplification) is inhibited, whereas amplification through the partial primer 2 (control amplification) occurs efficiently, and thus a difference between the calculated Ct values (ΔCt) of the two amplification curves may appear large. On the other hand, if the G13D mutation of the KRAS gene is present in the sample, amplification through both the partial primer 1 and the partial primer 2 occurs efficiently, and thus a difference between the calculated Ct values of the two amplification curves may appear small, thereby making it possible to easily determine the presence of the G13D mutation.

### 2.2 Production and verification of amplicon

In order to confirm the detection limit of G13D mutation when the clamping probe was not injected, a PCR composition including 10 pmoles of guide probe 1, 15 pmoles of guide probe 2, 10 pmoles of partial primer 1, 4 pmoles of partial primer 2, 4 pmoles of specific primer 1, 2 pmoles of specific primer 2, 3 pmoles of detection probe 1, and 3 pmoles of detection probe 2 was prepared to form the intended amplicon under conditions in which about 10,000 copies of a wild-type KRAS gene target nucleic acid were present, conditions in which about 10,000 copies of a wild-type KRAS gene target nucleic acid and about 1,000 copies of a KRAS gene target nucleic acid with G13D mutation were mixed (10%), conditions in which about 10,000 copies of a wild-type KRAS gene target nucleic acid and about 500 copies of a KRAS gene target nucleic acid with G13D mutation were mixed (5%), conditions in which about 10,000 copies of a wild-type KRAS gene target nucleic acid and about 100 copies of a KRAS gene target nucleic acid with G13D mutation were mixed (1%), conditions in which about 10,000 copies of a wild-type KRAS gene target nucleic acid and about 50 copies of a KRAS gene target nucleic acid with G13D mutation were mixed (0.5%), conditions in which about 10,000 copies of a wild-type KRAS gene target nucleic acid and about 25 copies of a KRAS gene target nucleic acid with G13D mutation were mixed (0.25%), or conditions in which about 10,000 copies of a wild-type KRAS gene target nucleic acid and about 10 copies of a KRAS gene target nucleic acid with G13D mutation were mixed (0.1%).

The PCR composition included not only a nucleic acid polymerase (DNA polymerase) for typical use in PCR but also components such as a buffer, deoxyribonucleotide-5-triphosphate (dNTP), potassium chloride (KCl), magnesium chloride (MgCl₂), a surfactant, etc.

Temperature control for PCR was performed using a general thermal cycler, and after initial denaturation [95°C, 5 minutes], as a first stage of 15 cycles, denaturation [95°C, 20 seconds] - annealing [63°C, 20 seconds] - extension [72°C, 20 seconds] was performed, after which as a second stage of 35 cycles, denaturation [95°C, 10 seconds] - measurement [50°C, 10 seconds] - denaturation [95°C, 20 seconds] - annealing [58°C, 20 seconds] - extension [72°C, 20 seconds] was performed. In the measurement step of the second stage, amplification curves were created by measuring the fluorescence values of the FAM and HEX channels at every cycle.

Then, the Ct value was calculated from the amplification curve obtained from the FAM channel and called "target region amplification Ct", and the Ct value was calculated from the amplification curve obtained from the HEX channel and called "control region amplification Ct". Based on results of obtaining the ΔCt value by subtracting the target region amplification Ct from the control region amplification Ct, a clear ΔCt difference was confirmed between the condition with 0.5 to 10% of G13D mutation and the condition with only the wild-type. When 0.1 to 0.25% of G13D mutation was injected, in some reactions, a ΔCt value similar to the condition with only the wild-type appeared, making it difficult to distinguish a desired mutation. Therefore, the detection limit under the condition in which the clamping probe was not injected was determined to be 0.5%.

Meanwhile, in order to compare the results of detection of G13D through an additional experiment when the clamping probe was injected, a PCR composition including 10 pmoles of guide probe 1, 15 pmoles of guide probe 2, 5 pmoles of clamping probe 1, 10 pmoles of partial primer 1, 4 pmoles of partial primer 2, 4 pmoles of specific primer 1, 2 pmoles of specific primer 2, 3 pmoles of detection probe 1, and 3 pmoles of detection probe 2 was prepared to form the intended amplicon under conditions in which about 10,000 copies of a wild-type KRAS gene target nucleic acid were present, conditions in which about 10,000 copies of a wild-type KRAS gene target nucleic acid and about 1,000 copies of a KRAS gene target nucleic acid with G13D mutation were mixed (10%), conditions in which about 10,000 copies of a wild-type KRAS gene target nucleic acid and about 500 copies of a KRAS gene target nucleic acid with G13D mutation were mixed (5%), conditions in which about 10,000 copies of a wild-type KRAS gene target nucleic acid and about 100 copies of a KRAS gene target nucleic acid with G13D mutation were mixed (1%), conditions in which about 10,000 copies of a wild-type KRAS gene target nucleic acid and about 50 copies of a KRAS gene target nucleic acid with G13D mutation were mixed (0.5%), conditions in which about 10,000 copies of a wild-type KRAS gene target nucleic acid and about 25 copies of a KRAS gene target nucleic acid with G13D mutation were mixed (0.25%), or conditions in which about 10,000 copies of a wild-type KRAS gene target nucleic acid and about 10 copies of a KRAS gene target nucleic acid with G13D mutation were mixed (0.1%).

The PCR composition included not only a nucleic acid polymerase (DNA polymerase) for typical use in PCR but also components such as a buffer, deoxyribonucleotide-5-triphosphate (dNTP), potassium chloride (KCl), magnesium chloride (MgCl₂), a surfactant, etc.

Temperature control for PCR was performed using a general thermal cycler, and after initial denaturation [95°C, 5 minutes], as a first stage of 15 cycles, denaturation [95°C, 20 seconds] - annealing [63°C, 20 seconds] - extension [72°C, 20 seconds] was performed, after which as a second stage of 35 cycles, denaturation [95°C, 10 seconds] - measurement [50°C, 10 seconds] - denaturation [95°C, 20 seconds] - annealing [58°C, 20 seconds] - extension [72°C, 20 seconds] was performed. In the measurement step of the second stage, amplification curves were created by measuring the fluorescence values of the FAM and HEX channels at every cycle.

Thereafter, the Ct value was calculated from the amplification curve obtained from the FAM channel and called "target region amplification Ct", and the Ct value was calculated from the amplification curve obtained from the HEX channel and called "control region amplification Ct". When the amplification curve did not rise and the Ct value could not be calculated, the Ct value was arbitrarily set to 35 with reference to the number of cycles in the second stage of temperature control for PCR.

Based on results of obtaining the ΔCt value by subtracting the target region amplification Ct from the control region amplification Ct, a clear ΔCt difference was confirmed between the condition with G13D mutation and the condition with only the wild-type. Specifically, the ΔCt value was -10 or more under all conditions (0.1 to 10%) with G13D mutation, whereas the ΔCt value was less than -20 when only the wild-type was injected, enabling a clear distinction. Therefore, the detection limit under the condition in which the clamping probe was injected was determined to be 0.1% or less.

Consequently, in the method of amplifying the target mutation nucleic acid using the PCR composition including the guide probe, the clamping probe, the partial primer, and the specific primer, high sensitivity that enables detection of the target nucleic acid present at a low concentration of 0.1% or in a very small amount corresponding to about 10 copies per reaction was confirmed. In particular, it was confirmed that amplification of a non-target wild-type nucleic acid was more effectively inhibited through injection of the clamping probe, resulting in increased sensitivity (FIGs. 5 and 6).

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Industrial Applicability]

According to the present invention, a method of amplifying a target nucleic acid is advantageous because a target nucleic acid present at a very low concentration can be amplified using a guide probe that binds to all hybridizable nucleic acids present in a sample and helps a partial primer bind to the detection region of the target nucleic acid, and also because nucleic acids other than the target nucleic acid differ in amplification rate due to the sequence specificity of the partial primer, and binding of the partial primer to nucleic acids other than the target nucleic acid is prevented by a clamping probe, whereby the target nucleic acid can be detected with high specificity. Therefore, the method of the present invention is useful for molecular diagnosis, prenatal diagnosis, early diagnosis, cancer diagnosis, genetic diagnosis, genotypic diagnosis, diagnosis of infectious bacteria, determination of drug-resistant bacteria, forensic medicine, identification of biological species, and the like.

### [Sequence List Free Text]

An electronic file is attached.

## Claims

1. A method of amplifying a target nucleic acid, comprising:
(a) amplifying an isolated nucleic acid by performing polymerase chain reaction (PCR) in presence of:
i) a guide probe comprising a sequence capable of hybridizing with a region of a target nucleic acid other than a detection region and a sequence not hybridizing with the target nucleic acid;
ii) a partial primer comprising a sequence complementary to the sequence not hybridizing with the target nucleic acid in the guide probe and a sequence complementary to the detection region of the target nucleic acid;
iii) a clamping probe that inhibits amplification of other nucleic acids except the target nucleic acid; and
iv) a specific primer capable of amplifying the target nucleic acid by pairing with the partial primer; and
(b) determining presence or absence of an amplicon.

2. The method according to claim 1, wherein the guide probe is configured such that the sequence hybridizing with the target nucleic acid is present at a 5' end or N-terminus and the sequence not hybridizing with the target nucleic acid is present at a 3' end or C-terminus.

3. The method according to claim 1, wherein the guide probe further comprises a linker between the sequence hybridizing with the target nucleic acid and the sequence not hybridizing with the target nucleic acid.

4. The method according to claim 3, wherein the linker is at least one selected from the group consisting of beta-alanine (β-Ala-OH, C3), aminobutyric acid (C4), aminohexanoic acid (C6), aminolauric acid (C12), acetoacetoxyethyl acrylate (AAEA (O-linker)), 2-[2-[2-[2-(amino)ethoxy]ethoxy]ethoxy]acetic acid (AEEEA), AEEEEA, DL15, and L35.

5. The method according to claim 1, wherein the guide probe, the partial primer, the clamping probe, and the specific primer comprise any one or a combination of two or more selected from among an oligonucleotide, LNA (locked nucleic acid), and PNA (peptide nucleic acid).

6. The method according to claim 5, wherein the guide probe is PNA with a length of 10 to 500 bases.

7. The method according to claim 6, wherein the guide probe is PNA with a length of 20 to 150 bases.

8. The method according to claim 1, wherein the guide probe and the clamping probe hybridize to a strand of the target nucleic acid opposite a strand to which the specific primer binds.

9. The method according to claim 1, wherein the partial primer further comprises a spacer, which is a single-stranded oligonucleotide with a length of 1 to 100 bases, between the sequence complementary to the sequence not hybridizing with the target nucleic acid in the guide probe and the sequence complementary to the detection region of the target nucleic acid.

10. The method according to claim 1, wherein the sequence complementary to the detection region of the target nucleic acid in the partial primer is a sequence of 3 to 15 bases.

11. The method according to claim 1, wherein the clamping probe is PNA with a length of 5 to 500 bases.

12. The method according to claim 1, wherein the clamping probe inhibits amplification of the other nucleic acids except the target nucleic acid by preventing the partial primer from binding to the other nucleic acids except the target nucleic acid.

13. The method according to claim 12, wherein the clamping probe comprises the sequence complementary to the detection region of the target nucleic acid in the partial primer, which differs by 1 to 10 bases.

14. The method according to claim 1, wherein the amplicon is 50 bp to 1 kbp in length.

15. The method according to claim 1, wherein determining the presence or absence of the amplicon in step (b) uses a nucleic acid-binding dye or probe capable of binding to the amplicon.

16. The method according to claim 15, wherein the nucleic acid-binding dye is selected from the group consisting of ethidium bromide, SYBR^{®} Green I, SYBR^{®} Gold, EvaGreen, YO-PRO-1, SYTO, BEBO, and BEXTO.

17. The method according to claim 15, wherein the probe capable of binding to the amplicon is selected from the group consisting of any one or a combination of two or more selected from among an oligonucleotide, LNA, and PNA.

18. The method according to claim 17, wherein the probe capable of binding to the amplicon has a reporter and a quencher connected to both ends thereof.

19. The method according to claim 18, wherein the reporter is at least one fluorescent material selected from the group consisting of fluorescein, fluorescein chlorotriazinyl, Rhodamine Green, Rhodamine Red, tetramethylrhodamine, FITC, Oregon Green, Alexa Fluor, FAM, JOE, ROX, HEX, Texas Red, TET, TRITC, TAMRA, cyanine-based dyes, and thiadicarbocyanine dyes.

20. The method according to claim 18, wherein the quencher is at least one selected from the group consisting of Dabcyl, TAMRA, Eclipse, DDQ, QSY, Blackberry Quencher, Black Hole Quencher, Qxl, Iowa Black FQ, Iowa Black RQ, and IRDye QC-1.

21. The method according to claim 1, wherein the isolated nucleic acid is isolated from a sample of a subject.

22. A PCR composition for amplifying a target nucleic acid, comprising:
i) a guide probe comprising a sequence capable of hybridizing with a region of a target nucleic acid other than a detection region and a sequence not hybridizing with the target nucleic acid;
ii) a partial primer comprising a sequence complementary to the sequence not hybridizing with the target nucleic acid in the guide probe and a sequence complementary to the detection region of the target nucleic acid;
iii) a clamping probe that inhibits amplification of other nucleic acids except the target nucleic acid; and
iv) a specific primer capable of amplifying the target nucleic acid by pairing with the partial primer.
